# EUROPEAN PATENT APPLICATION

(11) **EP 4 641 193 A1**
(43) Date of publication of application: **29.10.2025**
(21) Application number: 25165129.5
(22) Date of filing: 20.03.2025
(51) Int. Cl.: G01N 33/00, G01N 25/18, G01N 25/48

(54) **SELF-CALIBRATING HYDROGEN SENSOR**

(30) Priority: 26.04.2024 IN 202411033307
(71) Applicant: Honeywell International Inc., Charlotte, NC 28202 (US)
(72) Inventor: KUMAR, Nirmal A., Charlotte, 28202 (US); MARSH, Brian John, Charlotte, 28202 (US); MCCOLLUM, David, Charlotte, 28202 (US); OLSON, Thomas John, Charlotte, 28202 (US)
(74) Representative: Haseltine Lake Kempner LLP

(57) **Abstract**

A system is disclosed. The system comprises at least one thermal conductivity detector (TCD) configured to detect thermal conductivity in an ambient medium. Further, one or more sensors are configured to detect one or more inputs related to environmental conditions. Further, at least one hydrogen sensor is configured to detect presence of hydrogen in a sense medium. Further, one or more processors operationally coupled to at least one TCD, the one or more sensors, and at least one hydrogen sensors, are configured to determine a compensated thermal conductivity (TC) based on the one or more inputs received from the one or more sensors and the detected thermal conductivity from the at least one TCD; determine an estimated thermal conductivity (TC) based on the one or more inputs received from the one or more sensors; and determine a drift based at least on the compensated TC and estimated TC.

## Description

### TECHNOLOGICAL FIELD

Example embodiments of the present disclosure relate generally to a sensing device, and more particularly, to a self-calibrating hydrogen sensor.

### BACKGROUND

Hydrogen sensors are gas detectors that detect the presence of hydrogen. The hydrogen sensors are used to detect hydrogen leaks and are considered low cost, compact, durable, and easy to maintain as compared to other gas detecting sensing elements. Similarly, a thermal conductivity detector (TCD) hydrogen sensor is a type of gas sensor that detects the presence of hydrogen gas by measuring thermal conductivity of the hydrogen gas. Thermal conductivity detector (TCD) based hydrogen gas sensors are widely used in various applications such as hydrogen fuel cells, hydrogen storage, and hydrogen production. However, these sensors show a drift in response after a long period of time that is due to the electronic aging of components or reference standards in the sensor, along with inaccurate measurements. Such drifts many result in false alarms for hydrogen leaks.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a summary of some example embodiments to provide a basic understanding of some aspects of the present disclosure. This summary is not an extensive overview and is intended to neither identify key or critical elements nor delineate the scope of such elements. It will also be appreciated that the scope of the disclosure encompasses many potential embodiments in addition to those here summarized, some of which will be further described in the detailed description that is presented later.

In an example embodiment, a system is disclosed. The system comprises at least one thermal conductivity detector (TCD) configured to detect thermal conductivity in an ambient medium. Further, one or more sensors are configured to detect one or more inputs related to environmental conditions. Further, at least one hydrogen sensor is configured to detect presence of hydrogen in a sense medium. Further, one or more processors are operationally coupled to the at least one TCD, the one or more sensors, and the at least one hydrogen sensor. The one or more processors are configured to determine a compensated thermal conductivity (TC) based on the one or more inputs received from the one or more sensors and the detected thermal conductivity from the at least one TCD; determine an estimated thermal conductivity (TC) based on the one or more inputs received from the one or more sensors; and determine a drift based at least on the compensated TC and the estimated TC. In some embodiments, the drift is determined based on a difference of the estimated TC from the compensated TC.

In some embodiments, the one or more sensors comprises a temperature sensor, a humidity sensor, and a pressure sensor. Further, the one or more inputs related to environmental conditions comprises at least temperature, humidity, and pressure.

In some embodiments, the one or more processors are configured to determine a drift compensated TC based at least on a difference of the determined drift from the compensated TC.

In some embodiments, the one or more processors are further configured to convert the determined drift compensated TC to hydrogen parts per million (ppm) using Artificial Intelligence (AI)/ Machine Learning (ML) or statistical techniques.

In some embodiments, the compensated TC corresponds to thermal conductivity by filtering out the effect of the one or more inputs related to the environmental conditions on the thermal conductivity. In some embodiments, the estimated TC corresponds to thermal conductivity determined based at least on the one or more inputs related to the environmental conditions in real time.

In some embodiments, the one or more processors are configured to determine whether the hydrogen is present in the sense medium using the at least one hydrogen sensor. The one or more processors are configured to activate drift calibration on determining the hydrogen is absent in the sense medium. In some embodiments, the drift calibration is activated at recurring time intervals.

In some embodiments, the sense medium corresponds to environment in which the system is exposed to different locations of a machinery. In some embodiments, the at least one TCD corresponds to a Micro-electromechanical system (MEMS) TCD.

In another example embodiment, a method is disclosed. The method comprises steps of determining, via one or more processors, a compensated thermal conductivity (TC) based on one or more inputs received from one or more sensors and thermal conductivity detected by at least one thermal conductivity detector (TCD); determining, via the one or more processors, an estimated thermal conductivity (TC) based on the one or more inputs received from the one or more sensors; and determining, via the one or more processors, a drift based at least on the compensated TC and the estimated TC.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a block diagram of a system to re-calibrate a hydrogen sensor in accordance with an example embodiment of the present disclosure;
FIG. 2 illustrates a functional block diagram of the system to re-calibrate a hydrogen sensor in accordance with an example embodiment of the present disclosure; and
FIG. 3 illustrates a flowchart of a method to re-calibrate the hydrogen sensor in accordance with an example embodiment of the present disclosure.

### DETAILED DESCRIPTION

Some embodiments will now be described more fully hereinafter with reference to the accompanying drawings, in which some, but not all, embodiments of the present disclosure are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of systems and methods for re-calibrating a hydrogen sensor. Embodiments may allow a thermal conductivity detector (TCD) to detect thermal conductivity. Embodiments may allow one or more sensors to detect a plurality of environmental conditions within a sensing medium. Embodiments may allow one or more processors to receive one or more inputs from the one or more sensors. Embodiments may allow the one or more processors to determine a compensated thermal conductivity (TC) based on the one or more inputs received from the one or more sensors and the detected thermal conductivity from the at least one TCD. Embodiments may allow the one or more processors to determine an estimated thermal conductivity (TC) based on the one or more inputs received from the one or more sensors. Embodiments may allow the one or more processors to determine a drift based at least on the compensated TC and the estimated TC.

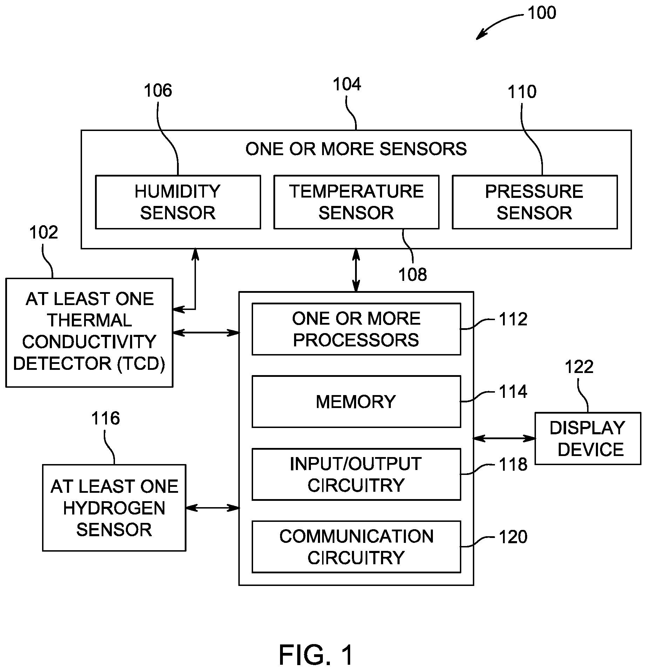

FIG. 1 illustrates a block diagram of a system 100 for re-calibrating a hydrogen sensor, in accordance with an example embodiment of the present disclosure. The system 100 may comprise at least one thermal conductivity detector (TCD) 102 and one or more sensors 104. The one or more sensors may comprise at least one of a humidity sensor 106, a temperature sensor 108, or a pressure sensor 110. The system 100 may comprises one or more processors 112 communicatively coupled to a memory 114. The system 100 may comprise at least one hydrogen sensor 116.

In some embodiments, the at least one TCD 102 may be configured to detect thermal conductivity in an ambient medium. In some embodiments, the ambient medium may correspond to the location or an environment where the system 100 may be positioned. Further, the one or more sensors 104 may be configured to determine one or more inputs related to environmental conditions. The environmental conditions may comprise at least one of humidity, temperature, or pressure. In some embodiments, the one or more sensors 104 may comprise at least one of the humidity sensor 106, the temperature sensor 108, or the pressure sensor 110. Further, the one or more processors 112 may be operationally coupled to the least one TCD 102 and the one or more sensors 104 to receive the thermal conductivity from the at least TCD 102 and the one or more inputs from the one or more sensors 104. The one or more processors 112 may receive the thermal conductivity from the at least TCD 102 and the one or more inputs from the one or more sensors 104 simultaneously.

In some embodiments, the at least one TCD 102 having a plurality of Thermal Conductivity Detectors (TCDs) utilizes a heating element. Further, each of the heating element may be composed of materials such as nichrome, tungsten, or platinum, that may be configured to generate heat when an electric current passes through, heating the surrounding air or gas. Further, the heated medium (sensing medium) may be configured to flow over the heating element and interact with analyte molecules present in a carrier gas stream. For gases like hydrogen, that may exhibit a high thermal conductivity, the presence of hydrogen molecules in the carrier gas alters the thermal conductivity of the medium. Further, the high thermal conductivity may leads to increased heat transfer rates compared to other gases. Further, the at least one TCD 102 may detect these changes in thermal conductivity by measuring temperature variations in the sensing medium. The presence of hydrogen or other thermally conductive gases may cause a distinct increase in the medium's temperature compared to non-conductive gases. Further, one or more downstream thermopiles consisting of multiple thermocouples may detect these temperature changes induced by variations in thermal conductivity. Further, the one or more downstream thermopiles may be configured to generate small electrical voltages proportional to the temperature changes, that may be processed to quantify the concentration of hydrogen or other thermally conductive gases in the sample.

Further, in MEMS-based TCDs, miniaturized components and integrated microfluidic pathways may be configured to enhance sensitivity and efficiency in detecting and analyzing hydrogen and other thermally conductive gases within compact devices. Further, the combination of precise heating, efficient gas interaction, and sensitive thermopile detection may create each of the TCDs suitable for applications requiring the detection and quantification of specific gases based on their thermal conductivity properties, including hydrogen detection in various gas mixtures.

In some embodiments, the one or more sensors 104 may be configured to detect one or more inputs related to environmental conditions. In some embodiments, the humidity sensor 106 may be operationally coupled with the one or more processors 112 to filter out the effect of humidity on the thermal conductivity. In some embodiments, the humidity sensor 106 may comprise one or more sensing elements (not shown) that changes its electrical properties in response to the humidity. The change in the electrical properties may be processed to determine the humidity in the air. Further, the pressure sensor 110 may comprise one or more pressure sensitive elements (not shown). The one or more pressure sensitive elements may convert a mechanical strain of pressure applied over the one or more pressure sensitive elements into one or more electrical signals. Further, the one or more electrical signals may be processed to determine the pressure of the air in the environmental conditions.

In some embodiments, the temperature sensor 108 may be composed of one or more metal probes (not shown). The one or more metal probes may generate an electrical voltage or resistance when the surrounding temperature change occurs by measuring the voltage across one or more diode terminals. In some embodiments, the change in voltage is directly proportional to change in temperature. The temperature sensor 108 may be configured to determine the electric signals caused due to change in temperature to determine the temperature in the environmental conditions. Further, the one or more inputs may be received from the one or more sensors 104, by the one or more processors 112. In some embodiments, the one or more processors 112 may analyze the received one or more inputs and the thermal conductivity to determine compensated thermal conductivity (TC). In some embodiments, the compensated TC may correspond to thermal conductivity by filtering out the effect of the one or more inputs related to the environmental conditions on the thermal conductivity. In some embodiments, a compensated Thermal Conductivity (TC) measurement may be configured to filter out environmental influences on thermal conductivity, ensuring accurate representation of intrinsic material properties. Further, the Mason-Saxena method involves analyzing the relationship between thermal conductivity and environmental factors like temperature, pressure, or humidity to develop a predictive model. Further, the Mason-Saxena method uses mathematical modeling and regression analysis to adjust raw thermal conductivity readings, providing more accurate data under standardized conditions. Other compensation approaches may involve experimental calibration using reference materials to establish baseline measurements for comparison and adjustment. Overall, these techniques enhance measurement accuracy by isolating and mitigating external influences on thermal conductivity, improving data reliability for analysis and interpretation.

Further, the one or more processors 112 may be configured to determine an estimated thermal conductivity (TC) based on the received one or more inputs from the one or more sensors 104. In some embodiments, the estimated TC may correspond to thermal conductivity determined based at least on the one or more inputs related to the environmental conditions (i.e., temperature, pressure, and humidity). The estimated TC may be determined in real time or in near real time with the receiving of the one or more inputs from the one or more sensors.

Further, the at least one hydrogen sensor 116 may be configured to detect presence of hydrogen in a sense medium. In some embodiments, the sense medium may correspond to an environment in which the system 100 is exposed. In some exemplary embodiment, the sense medium may be in proximity to the at least one TCD 102 and the one or more sensors 104. In some embodiments, the at least one hydrogen sensor 116 may correspond to a metal oxide semiconductor (MOS) based H2 sensor. Further, a Metal Oxide Semiconductor (MOS) hydrogen sensor may be configured to detect reversible changes in electrical resistance that may be caused by the adsorption and desorption of hydrogen gas (H2). The Metal Oxide Semiconductor (MOS) hydrogen sensor structure comprises a thin layer of metal oxide, such as silicon oxide (SiOx), situated between a metal electrode and a semiconductor substrate. Further, when exposed to hydrogen gas, H2 molecules may be adsorbed onto the surface of the metal oxide layer, altering its conductivity and increasing electrical resistance. Further, the increase in resistance is proportional to the concentration of hydrogen gas present, allowing for sensitive detection and quantification. As hydrogen gas is removed (desorbed) from the metal oxide layer, the resistance returns to its original state, enabling repeated measurements and real-time monitoring.

Electrical contacts connected to the metal and semiconductor layers of the sensor measure the changes in resistance, converting them into electrical signals for analysis and hydrogen gas concentration determination. Further, the at least one hydrogen sensor 116 may thereby determine the presence of hydrogen in the sense medium.

In one example embodiment, the TCD 102 and the one or more sensors 104 may be installed proximate to the at least one hydrogen sensor 116. For example, the TCD 102 and the one or more sensors 104 may be installed at a front portion underneath a hood of a vehicle. In another example embodiment, the TCD 102 and the one or more sensors 104 and the at least one hydrogen sensor 116 may be installed in different locations. For example, the TCD 102 and one or more sensors 104 may be installed underneath the hood of the vehicle and the at least one hydrogen sensor 116 may be installed inside a cabin of the vehicle.

Further, the one or more processors 112 may be configured to determine a drift based at least on the determined compensated TC and the estimated TC. In some embodiments, the one or more processors 112 may be configured to determine the drift only upon detecting the presence of hydrogen within the sense medium. Determining the drift only upon detecting the presence of hydrogen within the sense medium only when the hydrogen levels are below the levels normally occurring in air may be beneficial because it may be undesirable to determine drift when the presence of hydrogen is high, or the drift determinations may be inaccurate. Further, the memory 114 may store a set of instructions and data. In some embodiments, the memory 114 may include the one or more instructions that are executable by the one or more processors 112 to perform specific operations. It will be apparent to one skilled in the art that the one or more instructions stored in the memory 114 enable the hardware of the system 100 to perform the predetermined operations. Some of the commonly known memory 114 implementations include, but are not limited to, fixed (hard) drives, magnetic tape, floppy diskettes, optical disks, Compact Disc Read-Only Memories (CD-ROMs), and magneto-optical disks, semiconductor memories, such as ROMs, Random Access Memories (RAMs), Programmable Read-Only Memories (PROMs), Erasable PROMs (EPROMs), Electrically Erasable PROMs (EEPROMs), flash memory, magnetic or optical cards, or other type of media/machine-readable medium suitable for storing electronic instructions.

As illustrated in FIG. 1, the system 100 may comprise an input/output circuity 118, a communication circuitry 120, and a display device 122. In some embodiments, the input/output circuity 118 may enable a user to communicate or interface with the system 100. It may be noted that the input/output circuitry 118 may act as a medium to transmit input from the display device 122 to and from the system 100. In some embodiments, the input/output circuitry 118 may refer to the hardware and software components that facilitate the exchange of information between the user and the system 100. In one example, the display device 122 may include a graphical user interface (GUI) (not shown) as input/output circuitry 118 to allow the user to input data. The input/output circuitry 118 may include various input devices such as keyboards, barcode scanners, GUI for the user to provide data and various output devices such as displays, printers for the user to receive data.

In some embodiments, the system 100 may comprise the communication circuitry 120. The communication circuitry 120 may allow the system 100 and the display device 122 to exchange data or information with other systems or apparatuses. Further, the system 100 may be communicatively coupled with a network interface via one or more protocols and software modules for sending and receiving data or information. In some embodiments, the communication circuitry 120 may include Ethernet ports, Wi-Fi adapters, or communication protocols like HTTP or MQTT for connecting with other systems. The communication circuitry 120 may allow the system 100 to stay up-to-date. In some embodiments, the display device 122 may comprise at least one of mobile phones, laptops, or any other device known in the art.

It will be apparent to one skilled in the art that above-mentioned components of the system 100 have been provided only for illustration purposes, without departing from the scope of the disclosure.

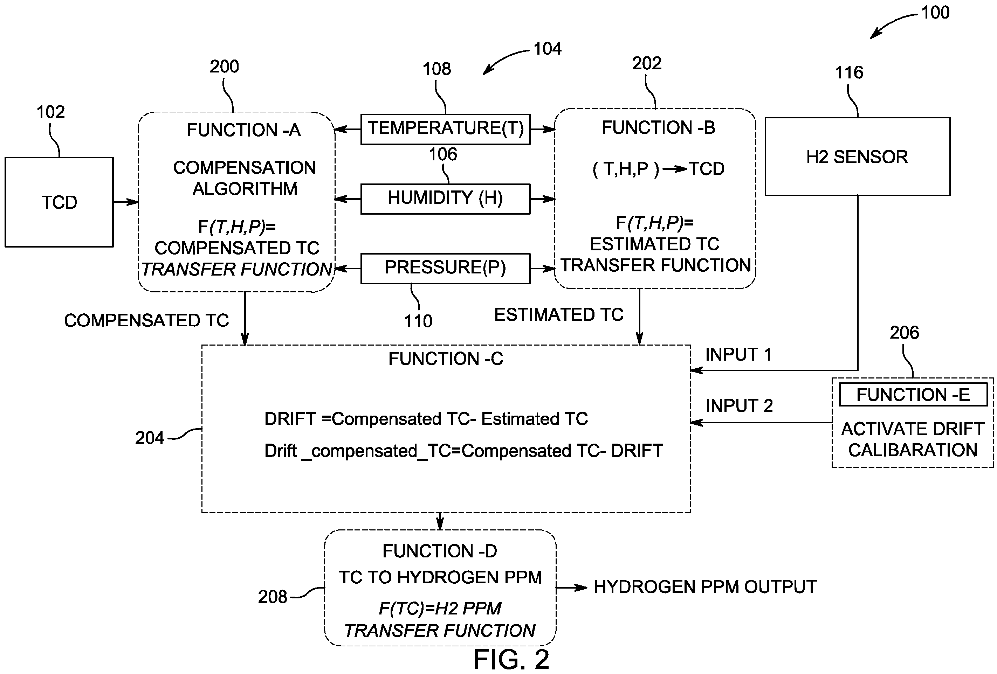

FIG. 2 illustrates a functional block diagram of the system 100 for re-calibrating a hydrogen sensor, in accordance with an example embodiment of the present disclosure.

In some embodiments, the at least one TCD 102 may be configured to detect thermal conductivity in the ambient medium. Further, the one or more sensors 104 may detect one or more inputs related to the environmental conditions. In some embodiments, the one or more processors 112 may be configured to receive the one or more inputs from the one or more sensors 104 and the detected thermal conductivity form the at least one TCD 102. In some embodiments, the one or more processors 112 may comprise a compensation module 200 denoted as FUNCTION - A. The compensation module 200 may be configured to determine compensated thermal conductivity (TC) based on the one or more inputs received from the one or more sensors 104 and the detected thermal conductivity from the at least one TCD 102.

In some embodiments, the compensation module 200 may be configured to filter out the effect of temperature, pressure and humidity on the thermal conductivity to determine the compensated TC using a transfer function. In some example embodiments, the transfer function may include polynomial functions, lookup tables, or other mathematical representations tailored to particular characteristics of compensated TC. In some embodiments, the compensation module 200 may be configured to use existing physics-based formula and equations developed using statistical or machine learning method.

In some embodiments, the one or more processors 112 may comprise a prediction model 202 denoted as FUNCTION-B. In some embodiments, the prediction model 202 may be configured to receive the one or more inputs from the one or more sensors 104 to predict or estimate approximate thermal conductivity at the current environmental conditions and provide an estimated TC. In some embodiments, the one or more processors 112 may be configured to further use the compensated TC and the estimated TC to determine the drift. In some embodiments, the one or more processors 112 may be configured to determine the drift only upon determining the presence of hydrogen within the sense medium via an INPUT 1 signal and upon receiving an INPUT 2 signal that indicates a request to activate a drift determination and/or calibration, as shown in FIG. 2.

In some embodiments, the one or more processors 112 may determine the presence of hydrogen in sense medium, via one or more inputs as denoted by INPUT 1, received from the at least one hydrogen sensor 116. In some embodiments, upon detecting the absence of the hydrogen, the INPUT 1 signal may be LOW and upon detecting the presence of the hydrogen, the INPUT 1 signal may be HIGH. Further, upon detecting a preset recurring time interval, the one or more processors 112 may be configured to generate the INPUT 2 signal as HIGH as shown in the FIG. 2 denoted as FUNCTION-E 206. Further, in case of not detecting the preset recurring time interval, the one or more processors 112 may be configured to generate the INPUT 2 signal as low.

Further, the one or more processors 112 analyzes the INPUT 1 signal and the INPUT 2 signal to activate drift calibration for determining the drift denoted as FUNCTION-C 204. The one or more processors 112 may be configured to determine the drift upon detecting the INPUT 1 signal as LOW and detecting the INPUT 2 signal as HIGH. In some embodiments, the drift calibration may be activated at recurring time intervals. In some embodiments, the one or more processors 112 may be configured to determine the drift based on a difference of the estimated TC from the compensated TC. Further, the one or more processors 112 may be configured to determine a drift compensated TC based at least on a difference of the determined drift from the compensated TC. Further, the one or more processors 112 may be configured to convert the determined drift compensated TC to hydrogen parts per million (ppm) using Artificial Intelligence (AI)/ Machine Learning (ML) or statistical techniques as denoted in FUNCTION-D208.

In some embodiments, the FUNCTION-D 208 may take output from the FUNCTION-C 204. Further, the one or more processors 112 may analyze the output of FUNCTION-C 204. In some embodiments, the output of FUNCTION-C 204 corresponds to drift compensated TC. In some embodiments, the one or more processors 112 may convert the drift compensated TC to hydrogen parts per million (ppm) by using transfer functions based on physics-based formula or equations developed using artificial intelligence/ Machine learning or statistical methods. In some embodiments, the output of FUNCTION-D 208 corresponds to Hydrogen ppm and may be an actual hydrogen concentration of the Metal oxide semiconductor (MOS) based H2 sensor 116.

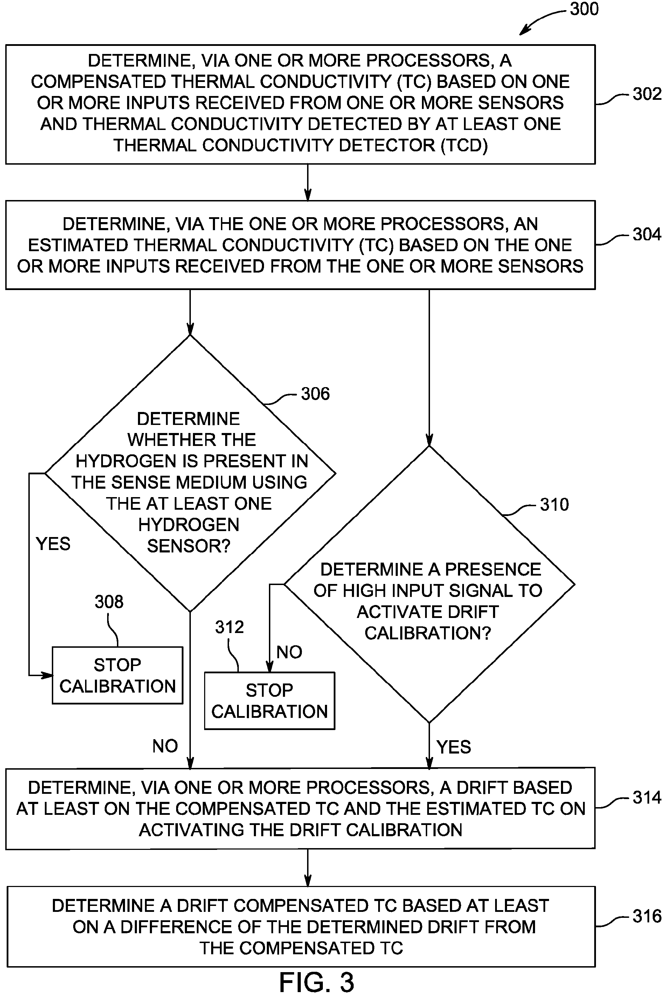

FIG. 3 illustrates a flowchart of a method 300 for re-calibrating a hydrogen sensor, in accordance with an example embodiment of the present disclosure. FIG. 3 is described in conjunction with FIGS. 1-2.

At operation 302, the one or more processors 112 may be configured to determine the compensated TC based on the one or more inputs received from one or more sensors 104 and thermal conductivity detected by the at least one TCD 102. In some embodiments, the one or more processors 112 may be configured to analyze the one or more inputs received from the at least one TCD 102 and the one or more sensors 104 to determine the compensated TC. In some embodiments, the compensated TC corresponds to thermal conductivity by filtering out the effect of the one or more inputs related to the environmental conditions on the thermal conductivity.

At operation 304, the one or more processors 112 are configured to determine an estimated TC based on the one or more inputs received from the one or more sensors 104. In some embodiments, the one or more processors 112 may estimate or predict the approximate thermal conductivity of the sense medium based at least on the current environmental conditions that is at least one of temperature, pressure, or humidity. In some embodiments, the estimated TC may correspond to the thermal conductivity determined based at least on the one or more inputs related to the environmental conditions in real time or in near real time.

At operation 306, the one or more processors 112 may be configured to determine whether the hydrogen is present in the sense medium using the at least one hydrogen sensor 116. In some embodiments, the one or more processors 112 may be configured to calculate the drift only upon determining the absence of hydrogen within the sense medium. In some embodiments, upon detecting the absence of the hydrogen, the one or more processors 112 may proceed at operation 314. Further, upon detecting the presence of the hydrogen, the one or more processors 112 may proceed at operation 308 to stop calibration.

At operation 310, the one or more processors 112 may be configured to determine a presence of high input signal for activating drift calibration. In some embodiments, the one or more processors 112 may be configured to activate the draft calibration at recurring time intervals. In some example embodiments, the recurring time intervals may be once per week and up to once every three years, such as once per year. In some embodiments, upon detecting the preset recurring time interval, the one or more processors 112 may be configured to generate the INPUT 2 signal as HIGH as shown in FIG. 2 denoted as FUNCTION-E 206. Further, in case of not detecting the preset recurring time interval, the one or more processors 112 may be configured to generate the INPUT 2 signal as low. The one or more processors 112 may determine the high input signal to proceed at step 314 to determine the drift. Further, upon determining a LOW input signal, the one or more processors 112 may direct at step 312 to stop the calibration. In some embodiments, the high input signal and the low input signal may be a mechanical, electrical, or digital signal that is supplied to the one or more processors 112.

At operation 314, the one or more processors 112 may be configured to determine drift based at least on the compensated TC and the estimated TC. In some embodiments, the one or more processors 112 may be configured to calculate the drift based on a difference of the estimated TC from the compensated TC.

At operation 316, the one or more processors 112 may be configured to determine the drift compensated TC based at least on a difference of the determined drift from the compensated TC. In some embodiments, the one or more processors 112 may further be configured to convert the determined drift compensated TC to hydrogen parts per million (ppm) using Artificial Intelligence (AI)/ Machine Learning (ML) or statistical techniques.

The present disclosure involves re-calibration of a hydrogen sensor that shows drift may lead to inaccurate measurements. The thermal conductivity may be determined by the TCD and the thermal conductivity is effected by humidity, temperature, pressure along with the presence of the hydrogen. The compensation algorithm filters out the effect of temperature, humidity, and pressure over the thermal conductivity, only in the absence of hydrogen. The compensation algorithm that are based on artificial intelligence (AI), machine learning (ML) and statistical methods are utilized to determine the drift of hydrogen sensor.

Many modifications and other embodiments of the disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A system comprising:
at least one thermal conductivity detector (TCD) configured to detect thermal conductivity in an ambient medium;
one or more sensors configured to detect one or more inputs related to environmental conditions;
at least one hydrogen sensor configured to detect presence of hydrogen in a sense medium; and
one or more processors operationally coupled to the at least one TCD, the one or more sensors, and the at least one hydrogen sensor, wherein the one or more processors are configured to:
determine a compensated thermal conductivity (TC) based on the one or more inputs received from the one or more sensors and the detected thermal conductivity from the at least one TCD;
determine an estimated thermal conductivity (TC) based on the one or more inputs received from the one or more sensors; and
determine a drift based at least on the compensated TC and the estimated TC.

2. The system of claim 1, wherein the one or more sensors comprises at least a temperature sensor, a humidity sensor, and a pressure sensor.

3. The system of claim 1, wherein the one or more inputs related to environmental conditions comprises at least one of temperature, pressure, or humidity.

4. The system of claim 1, wherein the drift is determined based on a difference of the estimated TC from the compensated TC.

5. The system of claim 1 further comprising determining a drift compensated TC based at least on a difference of the determined drift from the compensated TC.

6. The system of claim 5, wherein the one or more processors are further configured to convert the determined drift compensated TC to hydrogen parts per million (ppm) using Artificial Intelligence (AI)/ Machine Learning (ML) or statistical techniques.

7. The system of claim 1, wherein the compensated TC corresponds to thermal conductivity by filtering out the effect of the one or more inputs related to the environmental conditions on the thermal conductivity, and wherein the estimated TC corresponds to thermal conductivity determined based at least on the one or more inputs related to the environmental conditions in real time.

8. The system of claim 1 further comprising determining whether the hydrogen is present in the sense medium using the at least one hydrogen sensor.

9. The system of claim 8, wherein the one or more processors are configured to activate drift calibration on determining the hydrogen in the sense medium is absent in the sense medium and wherein the drift calibration is activated at recurring time intervals.

10. The system of claim 1, wherein the sense medium corresponds to environment in which the system is exposed to different locations of a machinery.

11. The system of claim 1, wherein the at least one TCD corresponds to a Micro-electromechanical system (MEMS) TCD.

12. A method comprising:
determining, via one or more processors, a compensated thermal conductivity (TC) based on one or more inputs received from one or more sensors and thermal conductivity detected by at least one thermal conductivity detector (TCD);
determining, via the one or more processors, an estimated thermal conductivity (TC) based on the one or more inputs received from the one or more sensors; and
determining, via the one or more processors, a drift based at least on the compensated TC and the estimated TC.

13. The method of claim 12, wherein the one or more sensors comprises at least a temperature sensor, a humidity sensor, and a pressure sensor.

14. The method of claim 12, wherein the one or more inputs related to environmental conditions comprises at least one of temperature, pressure, or humidity.

15. The method of claim 12, wherein the drift is determined based on a difference of the estimated TC from the compensated TC.
